## Europäisches Patentamt
## European Patent Office
## Office européen des brevets

(19)

(11) Publication number: **0 037 330**
**B1**

(12)

# EUROPEAN PATENT SPECIFICATION

(45) Date of publication of the patent specification:
07.03.84

(51) Int. Cl.³: **C 12 N 5/00,** C 07 G 7/00 //
A61K39/29

(21) Application number: 81400482.6

(22) Date of filing: 26.03.81

(54) Method of growing hepatitis B surface antigen.

(30) Priority: 27.03.80 US 134353

(43) Date of publication of application:
07.10.81 Bulletin 81/40

(45) Publication of the grant of the patent:
07.03.84 Bulletin 84/10

(84) Designated Contracting States:
BE DE FR GB IT NL SE

(56) References cited:
EP - A - 0 028 567

NATURE, vol. 282, December 6, 1979, Basingstoke, J. SKELLY et al. Hepatitis B surface antigen produced by a human hepatoma cell line" pages 617-618
BIOLOGICAL ABSTRACTS, vol. 63, no. 9, September 1977, abstr. 52500; Philadelphia, Pa, US, G.M. MACNAB et al. "Hepatitis B surface antigen produced by a human hepatoma cell line"

(73) Proprietor: MERCK & CO. INC., 126, East Lincoln Avenue P.O. Box 2000, Rahway New Jersey 07065 (US)

(72) Inventor: Keller, Paul M., 2057 Spring Valley Road, Lansdale Pennsylvania 19446 (US)
Inventor: McAleer, William J., 717 Marietta Drive, Ambler Pennsylvania 19002 (US)

(74) Representative: Corre, Jacques Denis Paul et al, Cabinet Regimbeau 26, Avenue Kléber, F-75116 Paris (FR)

## Method of growing hepatitis B surface antigen
## background of the invention

Hepatitis B surface antigen (HBsAg) has been shown to be effective as a vaccine against hepatitis B disease. The usual source of this antigen is plasma obtained from donors, e.g., by plasmaphoresis. As a result the supply of plasma containing this antigen is uncertain and expensive as most plasma is free of HBsAg.

Attempts have been made heretofore to grow HBsAg *in vitro*. For example, it is known that *in vitro* tissue cultures of human hepatoma cells produce HBsAg *in vitro* although in only trace amounts and with no detectable complement fixation titer. Humans infected with hepatitis B disease, on the other hand, produce large amounts of HBsAg — about $10^{13}$ particles/ml with a complement fixation titer of about 256.

### Objects of the invention

It is, accordingly, an object of the present invention to provide an *in vitro* tissue culture method for preparing HBsAg in high titer and purity. Another object is to provide an improved method for preparing HBsAg from human hepatoma cells which shed HBsAg. A further object is to provide a cell line cloned from human hepatoma cells which cell line produces HBsAg in high yield. These and other objects of the present invention will be apparent from the following description.

### Summary of the invention

Hepatitis B surface antigen is produced *in vitro* in high titer and purity from a cell line cloned from human hepatoma cells which shed HBsAg.

### Detailed description

The present invention relates to a method of growing hepatitis B surface antigen (HBsAg) *in vitro* from human hepatoma cells and, more particularly, to a method of growing HBsAg *in vitro* using a cell line cloned from human hepatoma tissue which has been found to shed HBsAg.

It has now been found that the yield of HBsAg increased markedly by using a cell line cloned from human hepatoma cells which shed HBsAg. The production of HBsAg by a human hepatoma cell line designated PLC/PRF/5, has been reported by Alexander *et al.*, S.A. Med. J. (1976) 2124-2128 and by NcNab *et al.*, Br. J. Cancer (1976) *34*, 509. However, any hepatoma cells which shed HBsAg can be used as starting material for the present invention. A large number of single cells from the hepatoma cell line are grown separately into colonies. The growth conditions preferably are substantially the same as those used to grow the starting hepatoma cell line. Typical conditions are those described by Alexander *et al.*, So. African J. Med. Sci. 41(2):89-98, 1976. Each colony is tested for production of HBsAg and consumption of glucose. Those colonies having the highest ratio of HBsAg production to glucose consumption are retained for HBsAg production. The yields of HBsAg may be measured by any suitable means. A radio-immune assay has been found to be suitable for this measurement although other assays, e.g., a complement fixation assay or an immune adherence hemagglutination assay, may also be used. The glucose assay may be measured by any suitable means. A glucose instrument analysis has been found to be suitable for this measurement although other assay suitable for measurement of glucose may also be used.

Whereas the starting cell line may have an HBsAg:glucose ratio of about 1.6:1, cloned colonies are obtained having an enhanced HBsAg:glucose ratio, typically a ratio of 4:1 or higher. By determining the ratio of HBsAg production and glucose consumption those clones which have lower HBsAg production are eliminated while those that have increased HBsAg production or lowered glucose consumption are selected. Those which have lowered glucose consumption but not increased antigen production are then eliminated by growing out the selected clones into bottles and testing for antigen produced per cell.

A culture of human hepatoma cell line PLC/PRF/5 has been deposited with American Type Culture Collection, Bethesda, Maryland and has been assigned accession number ATCC CRL 8024.

The following examples illustrate the present invention without, however, limiting the same thereon.

### Example 1:

Petri dishes, 60 mm diameter, are seeded with small pieces of glass cover slips (approximately 1-5 mm²). Cells from HBsAg-shedding hepatoma cell line PLC/PRF/5, suspended in EMEM growth media (Eagle Minimum Essential Medium) containing 10% FCS (fetal calf serum), are added to the petri dishes at approximately 5,000 cells/petri dish. This cell suspension has a ratio of HBsAg produced to glucose consumed of 1.6:1. The cells are allowed to attach to the glass cover slips. With the aid of a microscope about 200 cover slips with only one cell attached are removed and each is placed in an individual well of a multi-well assay plate filled with EMEM containing 10% FCS. The cells are allowed to grow into colonies for three to four weeks at 37°C in a 5% $CO_2$ atmosphere with refeeding after two weeks with the same growth medium. Each colony then is tested for consumption of glucose and production of HBsAg. The production of HBsAg is quantitated using a radio-immune assay for HBsAg giving a P/N ratio which corresponds to the antigen produced.

The colonies are compared on the basis of antigen produced to glucose consumed (P/N/glucose) per day. The colonies with the highest P/N/glucose ratio are grown out into bottles and retested after formation of a confluent cell sheet. These highest yielding clones produce about three to about eight times as much HBsAg as the starting PLC/PRF/5 cell line.

| Colony | HBsAg (P/N/ml) Production | Glucose (X10⁻² mg/ml) Consumption | P/N/ΔG Ratio |
|---|---|---|---|
| 1 | 281 | 36 | 7.8 |
| 2 | 212 | 48 | 4.42 |
| 3 | 13.9 | 01 | 13.9 |
| 4 | 74.3 | 19 | 3.91 |
| 5 | 179 | 46 | 3.9 |
| 6 | 173 | 36 | 4.9 |
| 7 | 146 | 31 | 4.7 |
| 8 | 126 | 26 | 4.9 |

Colony 3 although having the highest ratio fails to grow into a confluent monolayer and is eliminated. Colony 1 grows into a confluent monolayer and is selected for antigen production as it produces about six times as much antigen as the starting cell line. The remaining colonies are eliminated.

*Example 2:*

Twenty-four well cell culture plates, each well filled with 2 ml of growth media, EMEM (Eagle Minimum Essential Medium) containing 10% FCS (fetal calf serum), are seeded with 10,000 WI-38 cells which previously have been irradiated with 10,000 rad from a cobalt 60 source. A total of 2,500 wells are filled. After 48 hours at 37°C in a 5% $CO_2$ atmosphere, 0.2 ml of a suspension of a human hepatoma cell line (PLC/PRF/5) containing an average of 0.5 viable cells in 0.2 ml growth media is added to each of the wells.

The plated hepatoma cells are allowed to grow for four weeks with one 2 ml refeed of growth medium after 2 weeks. At the end of 4 weeks, wells with 1 visible hepatoma cell colony (approximately 550 colonies) are scored, refed with 2 ml of growth medium and stored at 32°C. Nine days later the medium from each colony is removed and tested for production of HBsAg and for consumption of glucose. The average P/N/Δ glucose ratio is approximately 7. The 8 colonies having the highest P/N/glucose ratio are grown out into bottles and retested after formation of a confluent cell sheet. These 8 colonies have the following ratios:

| Colony | P/N/ΔG |
|---|---|
| 1 | 23.0 |
| 2 | 23.0 |
| 3 | 29.9 |
| 4 | 35.0 |
| 5 | 37.3 |
| 6 | 43.2 |
| 7 | 50.0 |
| 8 | 72.0 |

## Claims

1. An *in vitro* method for preparing HBsAg in improved yield from a human hepatoma cell line which sheds HBsAg which comprises

cloning a human hepatoma cell line which sheds HBsAg;

selecting colonies having enhanced ratio of HBsAg production to glucose consumption; and

culturing the selected colonies under conditions which permit shedding of HBsAg.

2. A method according to claim 1 wherein the ratio of HBsAg production to glucose consumption is at least 3:1.

3. A method according to claim 2 wherein the ratio is about 6:1.

4. A method according to claim 1 wherein the cell line is ATCC CRL 8024.

5. A method according to claim 1 wherein the cloning is effected by means of a surface having attached thereto a single cell from the hepatoma cell line which sheds HBsAg.

6. A method according to claim 1 wherein the cloning is effected by adding to a receptacle in which cells are to be grown a cell suspension having an average of less than 1 cell per volume of cell suspension added to the receptacle.

## Revendications

1. Procédé *in vitro* pour préparer l'HBsAg avec un rendement amélioré à partir d'une lignée cellulaire d'hépatome humain excrétant l'HBsAg, dans lequel:

on clone une lignée cellulaire d'hépatome humain excrétant l'HBsAg;

on choisit des colonies ayant un rapport amélioré de production d'HBsAg à la consommation de glucose, et

on cultive les colonies choisies dans les conditions qui permettent l'excrétion d'HBsAg.

2. Procédé selon la revendication 1 où le rapport de la production d'HBsAg à la consommation de glucose est d'au moins 3:1.

3. Procédé selon la revendication 2 où le rapport est d'environ 6:1.

4. Procédé selon la revendication 1 où la lignée cellulaire est l'ATCC CRL 8024.

5. Procédé selon la revendication 1 où le clonage s'effectue au moyen d'une surface à laquelle est fixée une seule cellule de la lignée cellulaire d'hépatome excrétant l'HBsAg.

6. Procédé selon la revendication 1 où le clonage s'effectue en ajoutant à un réceptacle dans lequel on doit cultiver les cellules une suspension cellulaire ayant une moyenne inférieure à 1 cellule par volume de suspension cellulaire ajoutée au réceptacle.

## Patentansprüche

1. In vitro Verfahren zur Herstellung von HBsAg, in verbesserter Ausbeute, aus einer menschlichen Hepatom-Zellreihe, die HBsAg abgibt durch:

Klonen einer menschlichen Hepatom-Zellreihe, die HBsAg abigbt;

Auswählen von Kolonien mit einem erhöhten Verhältnis der HBsAg-Produktion zum Glucose-Verbrauch; und

Kultur der ausgewählten Kolonien unter Bedingungen, die die Abgabe von HBsAg erlauben.

2. Verfahren nach Anspruch 1, bei dem das Verhältnis der HBsAg-Produktion zum Glucose-Verbrauch mindestens 3:1 beträgt.

3. Verfahren nach Anspruch 2, bei dem das Verhältnis etwa 6:1 beträgt.

4. Verfahren nach Anspruch 1, bei dem die Zellreihe ATCC CRL 8024 ist.

5. Verfahren nach Anspruch 1, bei dem das Klonen mittels einer Oberfläche durchgeführt wird, an der eine einzelne Zelle aus der Hepatom-Zellreihe, die HBsAg abgibt, haftet.

6. Verfahren nach Anspruch 1, bei dem das Klonen durchgeführt wird, durch Einbringen in einen Aufnahmebehälter, in dem Zellen gezüchtet werden sollen, von einer Zellsuspension mit einem Durchschnitt von weniger als einer Zelle pro Volumen der Zellsuspension, die in den Aufnahmebehälter gefügt wird.